(19) Europäisches Patentamt
European Patent Office
Office européen des brevets

(11) Veröffentlichungsnummer: **0 413 244 A2**

# (12) EUROPÄISCHE PATENTANMELDUNG

(21) Anmeldenummer: **90115207.4**

(51) Int. Cl.[5]: **C07D 237/22**, A61K 31/50

(22) Anmeldetag: **08.08.90**

(30) Priorität: **16.08.89 DE 3926905**

(43) Veröffentlichungstag der Anmeldung:
**20.02.91 Patentblatt 91/08**

(84) Benannte Vertragsstaaten:
**BE CH DE DK ES FR GB IT LI NL**

(71) Anmelder: **BASF Aktiengesellschaft**
**Carl-Bosch-Strasse 38**
**D-6700 Ludwigshafen(DE)**

(72) Erfinder: **Trautmann, Walter, Dr.**
**Ritterbueschel 87**
**D-6730 Neustadt(DE)**
Erfinder: **Froehlich, Helmut, Dr.**
**Dirmsteiner Weg 35**
**D-6700 Ludwigshafen(DE)**
Erfinder: **Mildner, Wolfgang**
**Auf den Wergen 41**
**D-6734 Lambrecht(DE)**

## (54) Verfahren zur Abtrennung und Wiederverwertung von Reststoffen aus dem Abwasser der Synthese von 5-Amino-4-chlor-2-phenyl-3(2H)-pyridazinon.

(57) Verfahren zur Abtrennung und Wiederverwertung von Reststoffen aus dem Abwasser der Synthese von 5-Amino-4-chlor-2-phenyl-3(2H)-pyridazinon I

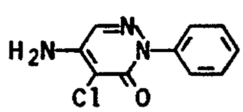

I

hergestellt aus 4,5-Dichlor-2-phenyl-3(2H)-pyridazinon II

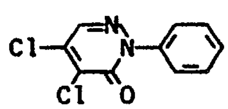

II

und Ammoniak, dadurch gekennzeichnet, daß man die im Abwasser gelösten Reste des Produktes I gemeinsam mit den Nebenprodukten
a) 4-Amino-5-chlor-2-phenyl-3(2H)-pyridazinon Ia

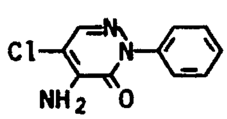

Ia

und
b) 4-chlor-5-hydroxy-2-phenyl-3(2H)-pyridazinon Ib

EP 0 413 244 A2

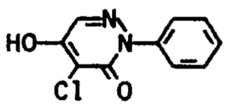

Ib

durch Ansäuern des Abwassers ausfällt und isoliert und das so erhaltene Gemisch mit einem Chlorierungsmittel in 4,5-Dichlor-2-phenyl-3(2H)-pyridazinon II überführt.

# VERFAHREN ZUR ABTRENNUNG UND WIEDERVERWERTUNG VON RESTSTOFFEN AUS DE ABWASSER DER SYNTHESE VON 5-AMINO-4-CHLOR-2-PHENYL-3(2H)-PYRIDAZINON

Die vorliegende Erfindung betrifft ein neues Verfahren zur Abtrennung und Wiederverwertung von Reststoffen aus dem Abwasser der Synthese von 5-Amino-4-chlor-2-phenyl-3(2H)-pyridazinon I,

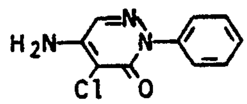

I

hergestellt aus 4,5-Dichlor-2-phenyl-3(2H)-pyridazinon II

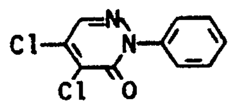

II

und Ammoniak.

Die Herstellung des herbiziden Wirkstoffs 5-Amino-4-chlor-2-phenyl-3-(2H)-pyridazinon I (Kurzbezeichnung Chloridazon) erfolgt nach bekannten Verfahren (DE-A 1 105 232, EP-A 26 847) durch Umsetzung von 4,5-Dichlor-2-phenyl-3(2H)-pyridazinon II mit wäßrigem Ammoniak entweder

a) unter Druck und bei erhöhter Temperatur, wobei man ein Gemisch aus ca. 80 Gew.% an 5-Amino-4-chlor-2-phenyl-3(2H)-pyridazinon I und ca. 20 Gew.% an 4-Amino-5-chlor-2-phenyl-3(2H)-pyridazinon Ia

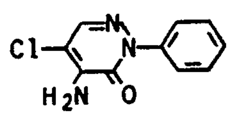

Ia

erhält, oder

b) unter Druck und bei erhöhter Temperatur in Gegenwart von Phenol-4-sulfonsäure, wobei man das gewünschte Produkt I in ca. 90 %iger Ausbeute und 95 bis 99 %iger Reinheit erhält.

Die Isolierung des 5-Amino-4-chlor-2-phenyl-3(2H)-pyridazinons I erfolgt in beiden Fällen durch Filtration des Festkörpers aus dem wäßrigen Reaktionsmedium. Der so erhaltene Feststoff wird anschließend mit Wasser nachgewaschen und getrocknet. Das wäßrige Filtrat und das Waschwasser fallen dabei als Abwasser an.

Gemäß Chem. Abstracts Vol. 68 (1968), 49555c, kann das Reaktionsnebenprodukt 5-Hydroxy-4-chlor-2-phenyl-3(2H)-pyridazinon Ib

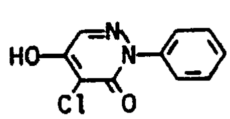

Ib

durch Ansäuern des Filtrats ausgefällt werden. Neben dem Reaktionsnebenprodukt Ib sind im Abwasser aber auch Reste der Verbindung Ia und des Produktes I enthalten.

Der Erfindung lag daher die Aufgabe zugrunde, die Verbindungen I, Ia und Ib aus dem Abwasser der vorstehend geschilderten Synthese von 5-Amino-4-chlor-2-phenyl-3(2H)-pyridazinon I zu entfernen und Möglichkeiten zur Wiederverwertung für diese Verbindungen zu finden.

Entsprechend dieser Aufgabe wurde ein Verfahren zur Abtrennung und Wiederverwertung von Reststoffen aus dem Abwasser der Synthese von 5-Amino-4-chlor-2-phenyl-3(2H)-pyridazinon I

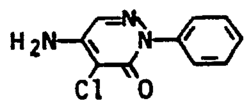

I

hergestellt aus 4,5-Dichlor-2-phenyl-3(2H)-pyridazinon II

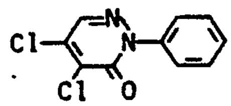

II

und Ammoniak, gefunden, das dadurch gekennzeichnet ist, daß man die im Abwasser gelösten Reste des Produktes I gemeinsam mit den Nebenprodukten

a) 4-Amino-5-chlor-2-phenyl-3(2H)-pyridazinon Ia

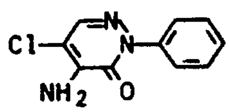

Ia

und

b) 4-Chlor-5-hydroxy-2-phenyl-3(2H)-pyridazinon Ib

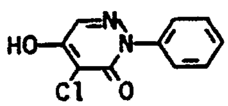

Ib

durch Ansäuern des Abwassers ausfällt und isoliert und das so erhaltene Gemisch bei Temperaturen von 70° C bis 140° C mit einem Chlorierungsmittel in 4,5-Dichlor-2-phenyl-3(2H)-pyridazinon II überführt.

Das Abwasser der Synthese von 5-Amino-4-chlor-2-phenyl-3(2H)-pyridazinon I hat üblicherweise einen pH-Wert von 7 bis 10. Sei Zugabe von Säure fallen die darin gelösten Pyridazinonderivate I, Ia und Ib als Feststoffe aus. Die Fällung beginnt etwa bei einem pH-Wert von ca. 5 und ist bei einem pH-Wert von 1 bis 2 vollständig. Ein pH-Wert von 1 sollte nicht unterschritten werden, da die Feststoffe dann unter Salzbildung wieder in Lösung gehen können.

Zum Ansäuern des Abwassers dienen bevorzugt Mineralsäuren wie Schwefelsäure, Salzsäure und Salpetersäure. Die Konzentration der eingesetzten Säure sollte, um die Abwassermenge nicht zusätzlich zu vergrößern, möglichst hoch sein. Dementsprechend wird die Mineralsäure bevorzugt mit Konzentrationen von 10 Gew.% bis 50 Gew.%, insbesondere 20 Gew.% bis 40 Gew.%, eingesetzt. Es ist beispielsweise auch möglich, Chlorwasserstoff in das Abwasser einzuleiten.

Der beim Ansäuern gebildete Feststoff aus den Pyridazinonderivaten I, Ia und Ib wird isoliert und getrocknet.

Eine Isolierung der Reststoffe aus dem Abwasser ist auch auf anderem Wege - beispielsweise durch Extraktion o.ä. möglich. Sie gestaltete sich jedoch im allgemeinen verfahrenstechnisch aufwendig bzw. führt im Beispiel der Extraktion zu einer zusätzlichen Belastung des Abwassers durch das z.T. gelöste Extraktionsmittel.

Zur Wiederverwertung der isolierten Reststoffe I, Ia und Ib wird der getrocknete und zerkleinerte Feststoff bei Temperaturen von 70° C bis 140° C, vorzugsweise 90° C bis 120° C mit einem Chlorierungsmittel in 4,5-Dichlor-2-phenyl-3(2H)-pyridazinon II überführt.

I

Ia

Ib

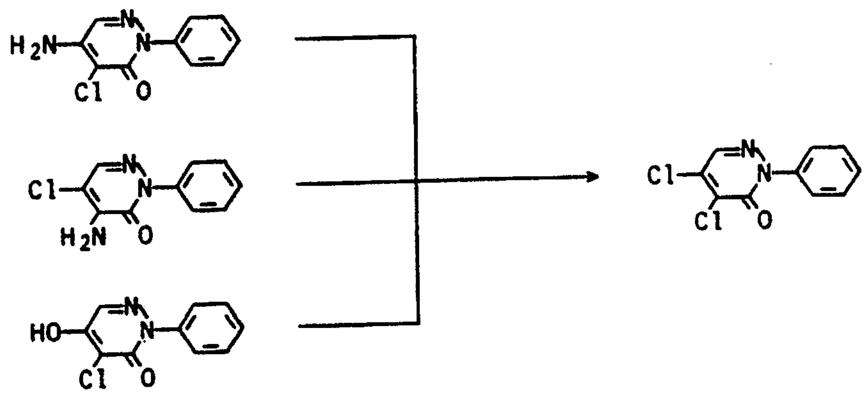

II

Geeignete Chlorierungsmittel sind Phosphortrichlorid, Phosphorpentachlorid und Thionylchlorid sowie insbesondere Phosphoroxychlorid.

Insbesondere dient das Chlorierungsmittel selbst in der 1- bis 20-fachen Gewichtsmenge, vorzugsweise 5 bis 10, bezogen auf den Feststoff als Lösungsmittel für den Feststoff.

Es kann in Abhängigkeit von der Art des Chlorierungsmittels vorteilhaft sein, diese Umsetzung bei erhöhtem Druck und einer entsprechend höheren Temperatur durchzuführen. Man arbeitet in diesen Fällen bei Drucken von 1 bis 30, vorzugsweise von 1 bis 15 bar und Temperaturen bis 200 °C.

Die Aufarbeitung der Reaktionsmischung erfolgt im allgemeinen durch destillative Entfernung des Chlorierungsmittels, Hydrolyse des Destillationsrückstands und Isolierung des dabei gebildeten festen Produkts. Das so gewonnene 4,5-Dichlor-2-phenyl-3(2H)-pyridazinon II kann ohne weitere Reinigung und ohne Trocknen zur Synthese von 5-Amino-4-chloro-2-phenyl-3(2H)-pyridazinon I verwendet werden.

Nach dem erfindungsgemäßen Verfahren zur Abtrennung und Wiederverwertung von Reststoffen aus der Synthese von 5-Amino-4-chloro-2-phenyl-3(2H)-pyridazinon I wird die Belastung der Reaktionsabwässer durch organische Substanzen verringert und die Wasseraufbereitung in Kläranlagen erleichtert. Das Verfahren gestattet außerdem auf elegante Weise die Wiederverwertung der wertvollen Pyridazinone.

Verfahrensbeispiel

1. Abtrennung der Reststoffe aus dem Abwasser

1000 g Abwasser aus der Synthese von 5-Amino-4-chlor-2-phenyl-3(2H)-pyridazinon (pH-Wert 8,8; Gehalt: 0,14 Gew.% I, 0,03 Gew.% Ia und 0,38 Gew.% Ib) wurden unter Kühlung mit 30 gew.%iger Salzsäure versetzt, bis ein pH-Wert von 1,5 erreicht war. Der dabei gebildete Feststoff wurde abfiltriert und getrocknet.

Man erhielt so 6,4 g eines Gemisches aus 15 Gew.% I, 2 Gew.% Ia und 60 Gew.% Ib (gemäß HPLC). Im sauren Filtrat verblieben 0,026 Gew.% I, 0,017 Gew.% Ia und 0,028 Gew.% Ib (gemäß HPLC-Analyse).

2. Wiederverwertung der Reststoffe

12 g des Feststoffs aus 1. (Gehalt gemäß HPLC: 15 Gew.% I, 2 Gew.% Ia und 60 Gew.% Ib) wurden gemeinsam mit 80 g Phosphoroxytrichlorid 10 h bei 90 bis 105 °C unter Rückfluß zum Sieden erhitzt. Anschließend wurde das überschüssige Phosphoroxytrichlorid bei vermindertem Druck (ca. 50 mbar) abdestilliert. Der Destillationsrückstand wurde bei 20 °C mit Wasser hydrolysiert, wobei 4,5-Dichlor-2-phenyl-3(2H)-pyridazinon als Feststoff ausfiel. Durch Filtration erhielt man 17,0 g feuchtes Produkt (Gehalt gemäß HPLC: 35,5 Gew.% 4,5-Dichlor-2-phenyl-3(2H)-pyridazinon und 44,7 Gew.% $H_2O$).

## Ansprüche

1. Verfahren zur Abtrennung und Wiederverwertung von Reststoffen aus dem Abwasser der Synthese von 5-Amino-4-chlor-2-phenyl-3(2H)-pyridazinon I

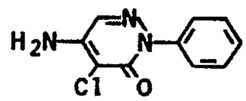

I

hergestellt aus 4,5-Dichlor-2-phenyl-3(2H)-pyridazinon II

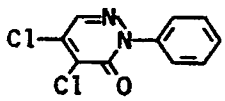

II

und Ammoniak, dadurch gekennzeichnet, daß man die im Abwasser gelösten Reste des Produktes I gemeinsam mit den Nebenprodukten

a) 4-Amino-5-chlor-2-phenyl-3(2H)-pyridazinon Ia

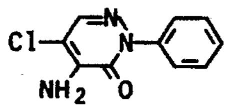

Ia

und

b) 4-Chlor-5-hydroxy-2-phenyl-3(2H)-pyridazinon Ib

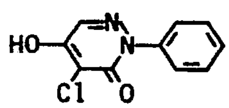

Ib

durch Ansäuern des Abwassers ausfällt und isoliert und das so erhaltene Gemisch mit einem Chlorierungsmittel in 4,5-Dichlor2-phenyl-3(2H)-pyridazinon II überführt.

2. Verfahren nach Anspruch 1, dadurch gekennzeichnet, daß man das Abwasser mit einer Mineralsäure auf einen pH-Wert unter 4 einstellt.

3. Verfahren nach Anspruch 1, dadurch gekennzeichnet, daß man die Chlorierung bei Temperaturen von 70 °C bis 140 °C mittels Phosphoroxychlorid vornimmt.

4. Verfahren nach Anspruch 3, dadurch gekennzeichnet, daß man die Chlorierung bei Temperaturen von 90 °C bis 120 °C durchführt.

5. Verfahren nach den Ansprüchen 1 bis 4, dadurch gekennzeichnet, daß man die Chlorierung bei Temperaturen von 90 °C bis 140 °C mittels Phosphoroxychlorid bei Drucken von 1 bar bis 30 bar vornimmt.